# EUROPEAN PATENT APPLICATION

(11) **EP 2 487 171 A1**
(43) Date of publication of application: **15.08.2012**
(21) Application number: 11153664.5
(22) Date of filing: 08.02.2011
(51) Int. Cl.: C07D 311/68, C07D 311/70, C07D 335/06, A61K 31/382, A61K 31/366, A61P 35/00

(54) **Dihydrobenzopyran, thiochroman, tetrahydroquinoleine and tetrahydronaphthalene derivatives and their use in anti-cancer therapy**

(71) Applicant: Université de Liège, 4031 Angleur (Liege) (BE); Université Libre de Bruxelles, 1050 Brussels (BE)
(72) Inventor: Goffin, Eric, 4000, Liege (BE); de Tullio, Pascal, 4000,Liege (BE); Florence, Xavier, 4000, Liege (BE); Pirotte, Bernard, 4000,Liege (BE); Rogister, Bernard, 4000, LIEGE (BE); Lamoral-Theys, Delphine, 1050, Bruxelles (BE); Kiss, Robert, 1050, Bruxelles (BE); Lefranc, Florence, 1050, Bruxelles (BE)

(57) **Abstract**

New dihydrobenzopyran, thiochroman, tetrahydroquinoleine and tetrahydronaphthalene derivatives and their use in anti-cancer therapy with the general formula :

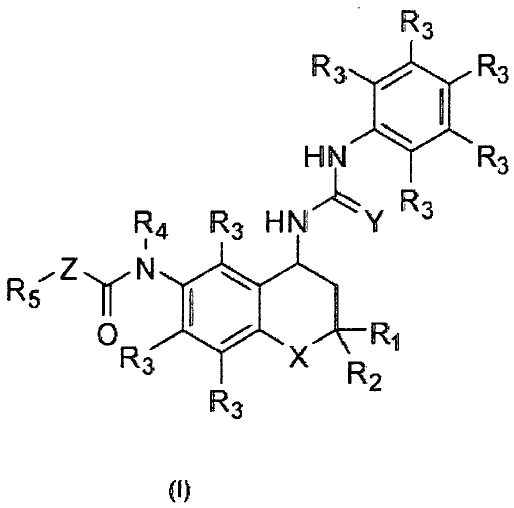

## Description

The present invention relates to new compounds, particularly dihydrobenzopyran, thiochroman, tetrahydroquinoleine and tetrahydronaphthalene derivatives, to pharmaceutical compositions comprising thereof and to their use for treatment of diseases, particularly proliferative disorder such as cancer.

### BACKGROUND OF THE INVENTION

Proliferative disorder such as cancer is becoming a more and more important cause of death amongst humans. More than 80% of all anti-cancer drugs are directed towards the apoptosis pathway of tumor cells and are cytotoxic upon activating said pathway. They are called antineoplastic agents, neoplastic referring to cancer cells. The very nature of antineoplastic agents makes them harmful to healthy constantly dividing cells and tissues, as well as the cancerous cells.

A large number of cancer cells such as glioblastomas (brain cancers), brain metastases, melanomas, pancreatic cancers, lung cancers of the NSCLC-type, refractory prostate cancers (HRPC), breast cancers such as triple negatives and other types are naturally resistant to apoptosis and cannot be treated by the many known drugs and chemotherapeutics.

The present invention therefore investigated the potential of new compounds, to have a cytotoxic and/or a cytostatic effect on apoptosis resistant tumor cells or cancer cells, especially in the context of brain cancers including gliomas.

There is a continuous need in the art for improving the efficacy of antiproliferative treatments in humans by providing suitable combinations of new drugs with conventional antineoplastic agents.

### SUMMARY OF THE INVENTION

We have now found new compounds particularly dihydrobenzopyran, thiochroman, tetrahydroquinoleine and tetrahydronaphthalene derivatives that provide a solution to the above stated problem.

We have unexpectedly found that the compounds of the invention appear to have important anti-cancer effects, while not impairing normal cell biology of non-tumor or non-cancer cells.

The new compounds are represented by general formula (I): 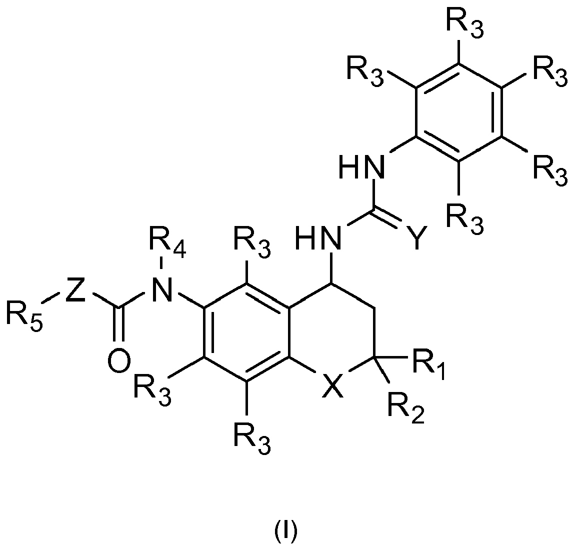 wherein
X represents Oxygen, Sulphur or an element selected from the group consisting of ( -NR₅, -CR₅R₆- or -SO₂,); wherein R₅ and R₆ which can be identical or different, denotes each independently one element selected from the group consisting of (hydrogen, a C₁₋₁₂-alkyl optionally mono- or polysubstituted with a halogen atom or a C₃₋₈-cycloalkyl optionally mono- or polysubstituted with a halogen atom) assuming that if Z represents Oxygen or Sulphur, R₅ cannot be hydrogen;
Y represents Oxygen or Sulphur
Z represents Oxygen or Sulphur or NH; if Z represents NH, then R₄ is different from H

R₁, R₂ and R₄ represent a hydrogen atom, a C₁₋₁₂-alkyl optionally mono- or polysubstituted with a halogen atom or a C₃₋₈-cycloalkyl group optionally mono- or polysubstituted with a halogen atom;
R₁ and R₂ optionally form together a hydrocarbon ring of 3 to 6 atoms, one carbon atom optionally being replaced by an oxygen atom.

R₃ represents an element selected from the group consisting of (hydrogen, halogen, C₁₋₁₂-alkyl optionally mono- or polysubstituted with a halogen atom, C₃₋₈-cycloalkyl optionally mono- or polysubstituted with a halogen atom, hydroxy, C₁₋₆-alkoxy, C₁₋₆-alkoxy-C₁₋₆-alkyl, nitro, amino, aminomethyl, cyano, cyanomethyl, perhalomethyl, C₁₋₆-monoalkyl- or dialkylamino, sulfamoyl, C₁₋₆-alkylthio, C₁₋₆-alkylsulfonyl, C₁₋₆-alkylsulfinyl, formyl, C₁₋₆-alkylcarbonylamino, R₇arylthio, R₇arylsulfinyl, R₇arylsulfonyl, C₁₋₆-alkoxycarbonyl, C₁₋₆-alkoxycarbonyl-C₁₋₆-alkyl, carbamoyl, carbamoylmethyl, C₁₋₆-monoalkyl- or dialkylaminocarbonyl, C₁₋₆-monoalkyl- or dialkylaminothiocarbonyl, ureido, C₁₋₆-monoalkyl- or dialkylaminocarbonylamino, thioureido, C₁₋₆-monoalkyl- or dialkylaminothiocarbonylamino, C₁₋₆-monoalkyl- or dialkylaminosulfonyl, carboxy, carboxy-C₁₋₆-alkyl, acyl, R₇aryl, R₇arylalkyl, R₇aryloxy),
where R₇ denotes one or several elements selected from the group consisting of (hydrogen, C₁₋₆-alkyl, halogen, hydroxy or C₁₋₆-alkoxy).

" C₁₋₆-alkyl" as used herein, alone or in combination, refers to a straight or branched, saturated hydrocarbon chain having 1 to 6 carbon atoms such as methyl, propyl, butyl, isopentyl, hexyl, 1-methylbutyl, 1,2-dimethylbutyl, 2-ethylbutyl, 2-methylpentyl, 3-methylpentyl and the like.

"C₁₋₁₂-alkyl" as used herein, alone or in combination, refers to a straight or branched, saturated hydrocarbon chain having 1 to 12 carbon atoms.

"C₃₋₈-cycloalkyl" as used herein refers to a radical of a saturated cyclic hydrocarbon chain having 3 to 8 carbon atoms such as cyclopropyl, cyclobutyl, cyclopentyl, cyclohexyl, and the like.

"C₁₋₆-alkoxy" as used herein, alone or in combination, refers to a straight or branched monovalent substituent comprising a C₁₋₆-alkyl group linked through an ether oxygen having its free valence bond from the ether oxygen and having 1 to 6 carbon atoms such as methoxy, ethoxy, propoxy, isopropoxy, butoxy, pentoxy, tertbutoxy and the like.

"C₁₋₆-alkoxy-C₁₋₆-alkyl" as used herein refers to a group of 2-12 carbon atoms interrupted by an oxygen atom such as -CH₂-O-CH₃, -CH₂CH₂O-CH₃, -CH₂-O-CH₂CH₃, -CH₂-O-CH(CH₃)₂, -CH₂CH₂-O-CH(CH₃)₂, -CH(CH₃)CH₂-O-CH₃ and the like.

"halogen" means fluorine, chlorine, bromine or iodine.

"perhalomethyl" means trifluoromethyl, trichloromethyl, tribromomethyl or triiodomethyl.

" C₁₋₆-monoalkylamino" as used herein refers to an amino group wherein one of the hydrogen atoms is substituted with a straight or branched, saturated hydrocarbon chain having 1 to 6 carbon atoms such as methylamino, ethylamino, propylamino, isopropylamino, butylamino, tert-butylamino, isopentylamino, hexylamino and the like.

" C₁₋₆-dialkylamino" as used herein refers to an amino group wherein the two hydrogen atoms independently are substituted with a straight or branched, saturated hydrocarbon chain having 1 to 6 carbon atoms such as dimethylamino, N-ethyl-N-methylamino, N-methyl-N-isopropylamino, N-butyl-N-methylamino, dihexylamino and the like.

"C₁₋₆-alkylthio" as used herein, alone or in combination, refers to a straight or branched monovalent substituent comprising a C₁₋₆-alkyl group linked through a divalent sulfur atom having its free valence bond from the sulfur atom and having 1 to 6 carbon atoms such as methylthio, ethylthio, propylthio, isopropylthio, butylthio, pentylthio, 3-methylpentylthio and the like.

"C₁₋₆-alkylsulfonyl" as used herein refers to a monovalent substituent comprising a C₁₋₆-alkyl group linked through a sulfonyl group (-S(=O)₂-) such as methylsulfonyl, ethylsulfonyl, propylsulfonyl, isopropylsulfonyl, butylsulfonyl, pentylsulfonyl, 2-methylpentylsulfonyl and the like.

"C₁₋₆-alkylsulfinyl" as used herein refers to a monovalent substituent comprising a C₁₋₆-alkyl group linked through a sulfinyl group (-S(=O)-) such as methylsulfinyl, ethylsulfinyl, propylsulfinyl, isopropylsulfinyl, tert-butylsulfinyl, pentylsulfinyl, 2-ethylbutylsulfinyl and the like.

"acyl" as used herein refers to a monovalent substituent comprising a C₁₋₆-alkyl group linked through a carbonyl group such as acetyl, propionyl, butyryl, isobutyryl, pivaloyl, valeryl and the like.

"C₁₋₆-alkylcarbonylamino" as used herein refers to an amino group wherein one of the hydrogen atoms is substituted with an acyl group such as acetamido, propionamido, isopropylcarbonylamino, 2-ethylbutylcarbonylamino and the like.

"aryl" as used herein refers to phenyl, 1-naphthyl, or 2-naphthyl.

"arylthio" as used herein, alone or in combination, refers to an aryl group linked through a divalent sulfur atom having its free valence bond from the sulfur atom, the aryl group is substituted or not by one or several elements of R₇ such as phenylthio, 1-naphthylthio, 2-methylphenylthio, 3-methoxyphenylthio and the like.

"arylsulfinyl" as used herein, alone or in combination, refers to an aryl group linked through a sulfinyl group (-S(=O)-), the aryl group is substituted or not by one or several elements of R₇ such as phenylsulfinyl, 2-methylphenylsulfinyl, 3-chloro-1-naphthylsulfinyl and the like.

"arylsulfonyl" as used herein, alone or in combination, refers to an aryl group linked through a sulfonyl group (-S(=O)₂-), the aryl group is substituted or not by one or several elements of R₇ such as phenylsulfonyl, 2-methylphenylsulfonyl, 4-iodophenylsulfonyl, 2-naphthylsulfonyl and the like.

"Cₗ-₆-alkoxycarbonyl" as used herein refers to a monovalent substituent comprising a C₁₋₆-alkoxy group linked through a carbonyl group such as methoxycarbonyl, isopropoxycarbonyl, butoxycarbonyl, tert-butoxycarbonyl, 2-methylpentoxycarbonyl and the like.

"C₁-₆-monoalkylaminocarbonyl" as used herein refers to a monovalent substituent comprising a C₁-₆-monoalkylamino group linked through a carbonyl group such as methylaminocarbonyl, isopropylaminocarbonyl, butylaminocarbonyl, 2-methylbutylaminocarbonyl and the like.

"C₁₋₆-dialkylaminocarbonyl" as used herein refers to a monovalent substituent comprising a C₁₋₆-dialkylamino group linked through a carbonyl group such as dimethylaminocarbonyl, diethylaminocarbonyl, N-methyl-N-isopropylaminocarbonyl, N-methyl-N-butylaminocarbonyl, N-propyl-N-2-methylbutylaminocarbonyl and the like.

"C₁₋₆-monoalkylaminothiocarbonyl" as used herein refers to a monovalent substituent comprising a C₁₋₆-monoalkylamino group linked through a thiocarbonyl group such as methylaminothiocarbonyl, isopropylaminothiocarbonyl, butylaminothiocarbonyl, 3-methylpentylaminothiocarbonyl, 1,2-dimethylbutylaminothiocarbonyl and the like.

"C₁₋₆-dialkylaminothiocarbonyl" as used herein refers to a monovalent substituent comprising a C₁₋₆-dialkylamino group linked through a thiocarbonyl group such as dimethylaminothiocarbonyl, diethylaminothiocarbonyl N-methyl-N-isopropylaminothiocarbonyl, N-methyl-N-butylaminothiocarbonyl N-tert-butyl-N-hexylaminothiocarbonyl and the like.

" C₁₋₆-monoalkylaminocarbonylamino" as used herein refers to an amino group wherein one of the hydrogen atoms is substituted with a C₁₋₆-monoalkylaminocarbonyl group such as methylaminocarbonylamino, ethylaminocarbonylamino, propylaminocarbonylamino, 3-methylbutylaminocarbonylamino, 1,2-dimethylbutylaminocarbonylamino and the like.

" C₁₋₆-dialkylaminocarbonylamino" as used herein refers to an amino group wherein one of the hydrogen atoms is substituted with a C₁₋₆-dialkylaminocarbonyl group such as dimethylaminocarbonylamino, diethylaminocarbonylamino, N-methyl-N-ethylaminocarbonylamino, N-methyl-N-isopropylaminocarbonylamino, N-propyl-N-pentylaminocarbonylamino and the like.

" C₁₋₆-monoalkylaminothiocarbonylamino" as used herein refers to an amino group wherein one of the hydrogen atoms is substituted with a C₁₋₆-monoalkylaminothiocarbonyl group such as methylaminothiocarbonylamino, ethylaminothiocarbonylamino, propylaminothiocarbonylamino, 3-methylpentylaminothiocarbonylamino and the like.

" C₁₋₆-dialkylaminothiocarbonylamino" as used herein refers to an amino group wherein one of the hydrogen atoms is substituted with a C₁₋₆-dialkylaminothiocarbonyl group such as dimethylaminothiocarbonylamino, diethylaminothiocarbonylamino, N-methyl-N-ethylaminothiocarbonylamino, N-methyl-N-propylaminothiocarbonylamino , N-isopropyl-N-hexylaminothiocarbonylamino, N-3-methylpentyl-N-pentylaminothiocarbonylamino and the like.

"C₁₋₆-monoalkylaminosulfonyl" as used herein refers to a monovalent substituent comprising a C₁₋₆-monoalkylamino group linked through a sulfonyl group such as methylaminosulfonyl, ethylaminosulfonyl, propylaminosulfonyl, hexylaminosulfonyl, tert-butylaminosulfonyl, 1,2-dimethylbutylaminosulfonyl and the like.

"C₁₋₆-dialkylaminosulfonyl" as used herein refers to a monovalent substituent comprising a C₁₋₆-dialkylamino group linked through a sulfonyl group such as dimethylaminosulfonyl, diethylaminosulfonyl, N-methyl-N-ethylaminosulfonyl N-methyl-N-propylaminosulfonyl, N-hexyl-N-3-methylbutylaminosulfonyl and the like.

"Carbamoyl " as used herein refers to NH₂CO-

"sulfamoyl" as used herein refers to NH₂-SO₂-

"ureido" as used herein means -NH-CO-NH₂.

"thioureido" as used herein means -NH-CS-NH₂.

"arylalkyl" as used herein refers to a straight or branched saturated carbon chain containing from 1 to 6 carbons substituted with an aromatic carbohydride. the aryl group is substituted or not by one or several elements of R₇.

"aryloxy" as used herein refers to phenoxy, 1-naphthyloxy or 2-naphthyloxy, the aryl group is substituted or not by one or several elements of R₇.

"R₇aryl as used herein refers to aryl substituted by R₇.

"dihydrobenzopyran derivatives " as used herein refers to the compounds of Formula (I) wherein -X- is represented by -O- (oxygen).
"thiochroman derivatives" as used herein refers to the compounds of Formula (I) wherein -X- is represented by -S- (sulphur) or -SO₂-,
"tetrahydroquinoleine derivatives as used herein refers to the compounds of Formula (I) wherein -X- is represented by -NR₅-, and
"tetrahydronaphthalene derivatives" as used herein refers to the compounds of Formula (I) wherein -X- is represented by -CR₅R₆-.

This invention also refers to all optical isomers of the compounds represented by formula (I), particularly to all optically active isomers of dihydrobenzopyran, thiochroman, tetrahydroquinoleine and tetrahydronaphthalene derivatives, and their mixtures including racemic mixtures thereof.

When one has an asymetrical carbon atom in the compound of general formula (I), the invention also refers to pure optical isomers than to racemic mixture.

The invention refers also to tautomeric forms of the compounds represented by formula (I), particularly to tautomeric forms of dihydrobenzopyran, thiochroman, tetrahydroquinoleine and tetrahydronaphthalene derivatives and to pharmacologically acceptable salts of said compounds.

By pharmacologically acceptable salts of the derivatives, one means pharmaceutically acceptable acid addition salts, pharmaceutically acceptable metal salts or optionally alkylated ammonium salts.

Preferred compounds of the Invention are:
R/S-*N*-2-chlorophenyl-*N*'-(6-*tert*-butoxycarbonylamino-3,4-dihydro-2*H*-1-benzopyran-4-yl)urea
R/S-*N*-3-chlorophenyl-*N'*-(6-*tert*-butoxycarbonylamino-3,4-dihydro-2*H*-1-benzopyran-4-yl)urea
R/S-*N*-4-chlorophenyl-*N'*-(6-*tert*-butoxycarbonylamino-3,4-dihydro-2*H*-1-benzopyran-4-yl)urea
R/S-*N*-2-cyanophenyl-*N'*-(6-*tert*-butoxycarbonylamino-3,4-dihydro-2*H*-1-benzopyran-4-yl)urea
R/S-*N*-3-cyanophenyl-*N'*-(6-*tert*-butoxycarbonylamino-3,4-dihydro-2*H*-1-benzopyran-4-yl)urea
R/S-*N*-4-cyanophenyl-*N'*-(6-*tert*-butoxycarbonylamino-3,4-dihydro-2*H*-1-benzopyran-4-yl)urea
R/S-*N*-3-cyanophenyl-*N'*-(6-*tert*-butoxycarbonylaminothiochroman-4-yl)urea
R/S-*N*-3-chlorophenyl-*N'*-(6-*tert*-butoxycarbonylaminothiochroman-4-yl)urea
R/S-*N*-4-chlorophenyl-*N'*-(6-*tert*-butoxycarbonylaminothiochroman-4-yl)urea
R/S-*N*-2-chlorophenyl-*N'*-(6-*tert*-butoxycarbonylaminothiochroman-4-yl)urea
R/S-*N*-4-cyanophenyl-*N*'-(6-*tert*-butoxycarbonylaminothiochroman-4-yl)urea
R/S-*N*-2-cyanophenyl-*N*'-(6-*tert*-butoxycarbonylaminothiochroman-4-yl)urea
R/S-*N*-4-cyanophenyl-*N'*-(6-*tert*-butoxycarbonylamino-1,1-dioxo-thiochroman-4-yl)urea
R/S-*N*-3-cyanophenyl-*N'*-(6-*tert*-butoxycarbonylamino-3,4-dihydro-2,2-dimethyl-2*H-*1-benzopyran-4-yl)urea
R/S-*N*-4-cyanophenyl-*N'*-(6-*tert*-butoxycarbonylamino-3,4-dihydro-2,2-dimethyl-2*H-*1-benzopyran-4-yl)urea
R/S-*N*-3-chlorophenyl-*N'*-(6-*tert*-butoxycarbonylamino-3,4-dihydro-2,2-dimethyl-2*H-*1-benzopyran-4-yl)urea
R/S-*N*-4-chlorophenyl-*N'*-(6-*tert*-butoxycarbonylamino-3,4-dihydro-2,2-dimethyl-2*H-*1-benzopyran-4-yl)urea
R/S-*N*-4-fluorophenyl-*N'*-(6-*tert*-butoxycarbonylamino-3,4-dihydro-2,2-dimethyl-2*H*-1-benzopyran-4-yl)urea
R/S-*N*-3-fluorophenyl-*N'*-(6-*tert*-butoxycarbonylamino-3,4-dihydro-2,2-dimethyl-2*H*-1-benzopyran-4-yl)urea
R/S-*N*-3-bromophenyl-*N*'-(6-*tert*-butoxycarbonylamino-3,4-dihydro-2,2-dimethyl-2*H-*1-benzopyran-4-yl)urea
R/S-*N*-3,5-difluorophenyl-*N*'-(6-*tert*-butoxycarbonylamino-3,4-dihydro-2,2-dimethyl-2*H*-1-benzopyran-4-yl)urea
R/S-*N*-4-nitrophenyl-*N*'-(6-*tert*-butoxycarbonylamino-3,4-dihydro-2,2-dimethyl-2*H*-1-benzopyran-4-yl)urea
R/S-*N*-3-nitrophenyl-*N*'-(6-*tert*-butoxycarbonylamino-3,4-dihydro-2,2-dimethyl-2*H*-1-benzopyran-4-yl)urea
R/S-*N*-4-trifluoromethylphenyl-*N*'-(6-*tert*-butoxycarbonylamino-3,4-dihydro-2,2-dimethyl-2*H*-1-benzopyran-4-yl)urea
R/S-*N*-3-trifluoromethylphenyl-*N*'-(6-*tert*-butoxycarbonylamino-3,4-dihydro-2,2-dimethyl-2*H*-1-benzopyran-4-yl)urea
R/S-*N*-3,5-dichlorophenyl-*N*'-(6-*tert*-butoxycarbonylamino-3,4-dihydro-2,2-dimethyl-2*H*-1-benzopyran-4-yl)urea
R/S-*N*-2-nitrophenyl-*N*'-(6-*tert*-butoxycarbonylamino-3,4-dihydro-2,2-dimethyl-2*H*-1-benzopyran-4-yl)urea
R/S-*N*-2-trifluoromethylphenyl-*N*'-(6-*tert*-butoxycarbonylamino-3,4-dihydro-2,2-dimethyl-2*H*-1-benzopyran-4-yl)urea
R/S-*N*-3,5-di(trifluoromethyl)phenyl-*N*'-(6-*tert*-butoxycarbonylamino-3,4-dihydro-2,2-dimethyl-2*H*-1-benzopyran-4-yl)urea
R/S-*N*-2-chloro-4-trifluoromethylphenyl-*N*'-(6-*tert*-butoxycarbonylamino-3,4-dihydro-2,2-dimethyl-2*H*-1-benzopyran-4-yl)urea
R/S-*N*-2-chloro-5-trifluoromethylphenyl-*N*'-(6-*tert*-butoxycarbonylamino-3,4-dihydro-2,2-dimethyl-2*H*-1-benzopyran-4-yl)urea
R/S-*N*-2-chloro-6-trifluoromethylphenyl-*N*'-(6-*tert*-butoxycarbonylamino-3,4-dihydro-2,2-dimethyl-2*H*-1-benzopyran-4-yl)urea
R/S-*N*-4-chloro-3-trifluoromethylphenyl-*N*'-(6-*tert*-butoxycarbonylamino-3,4-dihydro-2,2-dimethyl-2*H*-1-benzopyran-4-yl)urea
R/S-*N*-4-bromophenyl-*N*'-(6-*tert*-butoxycarbonylamino-3,4-dihydro-2,2-dimethyl-2*H-*1-benzopyran-4-yl)urea
R/S-*N*-2-chlorophenyl-*N*'-(6-*tert*-butoxycarbonylamino-3,4-dihydro-2,2-dimethyl-2*H-*1-benzopyran-4-yl)urea
R/S-*N*-2-cyanophenyl-*N*'-(6-*tert*-butoxycarbonylamino-3,4-dihydro-2,2-dimethyl-2*H-*1-benzopyran-4-yl)urea
R/S-*N*-4-chloro-2-trifluoromethylphenyl-*N*'-(6-*tert*-butoxycarbonylamino-3,4-dihydro-2,2-dimethyl-2*H*-1-benzopyran-4-yl)urea

The present invention also refers to compounds particularly dihydrobenzopyran, thiochroman, tetrahydroquinoleine and tetrahydronaphthalene derivatives or their combination for use as a medicament.

In another aspect the invention relates to compounds particularly to dihydrobenzopyran, thiochroman, tetrahydroquinoleine and tetrahydronaphthalene derivatives of the general formula (I ) or a pharmaceutically acceptable acid addition salt thereof for use as a therapeutically acceptable substance, preferably for use as a therapeutically acceptable substance in the treatment or prevention of proliferative disorder, particularly cancer and most particularly brain cancer.

Preferably, the compounds of the present invention are used in the treatment or prevention of brain cancer such as for example gliomas, primitive neuroectodermal tumors (PNETs) and brain metastases.

The compounds, particularly dihydrobenzopyran, thiochroman, tetrahydroquinoleine and tetrahydronaphthalene derivatives of formula (I) and combinations thereof can be formulated in compositions such as tablets, capsules or elixirs for oral administration, in sterile solutions or suspensions.

Accordingly, the invention also refers to a pharmaceutical composition comprising an effective amount of compounds particularly dihydrobenzopyran, thiochroman, tetrahydroquinoleine or tetrahydronaphthalene derivatives or a combination thereof with a pharmaceutical acceptable carrier.

The pharmaceutical composition according to the invention may be administered as a dose in a range from about 0.05 to 1000, preferably from about 0.1 to 500 and especially in the range from 50 to 200 mg per day.

The invention also relates to a process for the manufacture of a medicament, particularly to be used in the treatment of proliferative disorder, which process comprising bringing a dihydrobenzopyran, thiochroman, tetrahydroquinoleine or tetrahydronaphthalene derivative of formula (I) or a pharmaceutically acceptable salt thereof into a galenic dosage form.

In still another aspect, the present invention relates to a method for preparing the above mentioned compounds particularly the dihydrobenzopyran, thiochroman, tetrahydroquinoleine and tetrahydronaphthalene derivatives.

The method comprises the following steps:

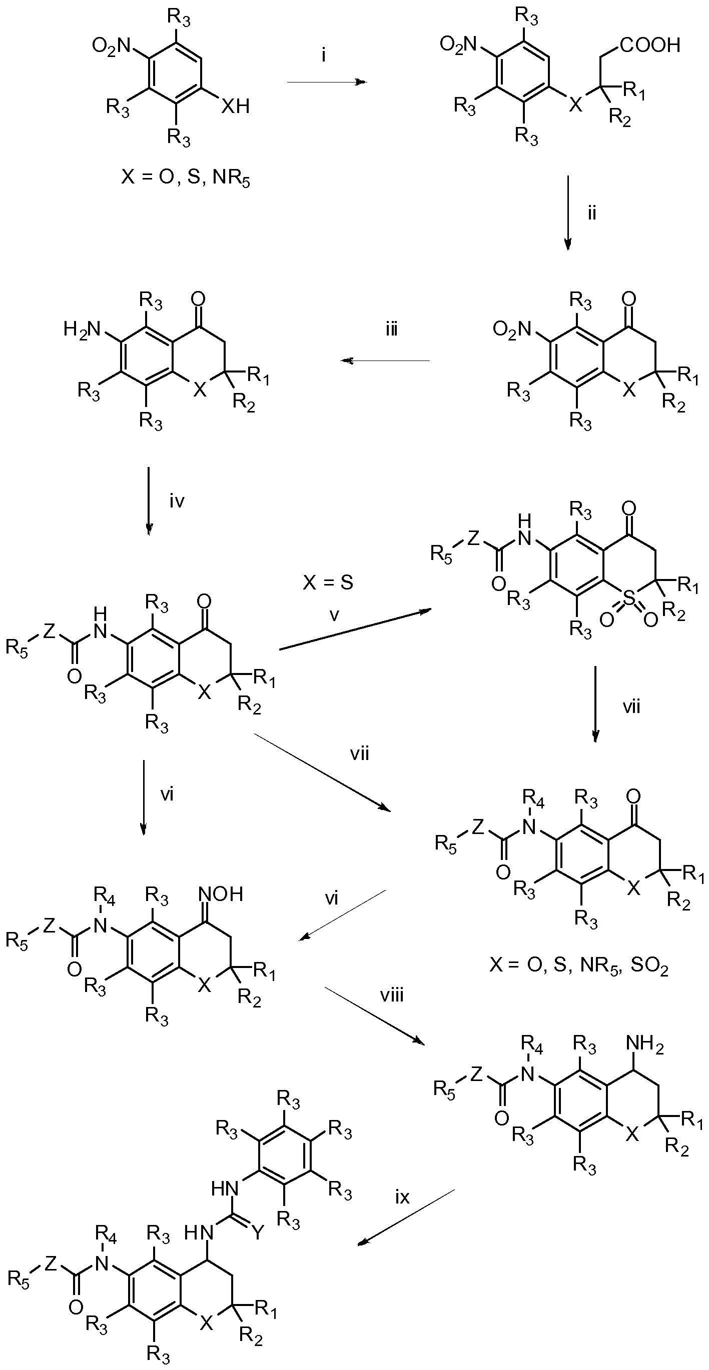

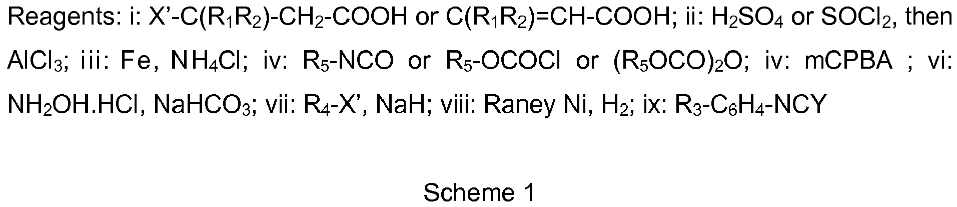

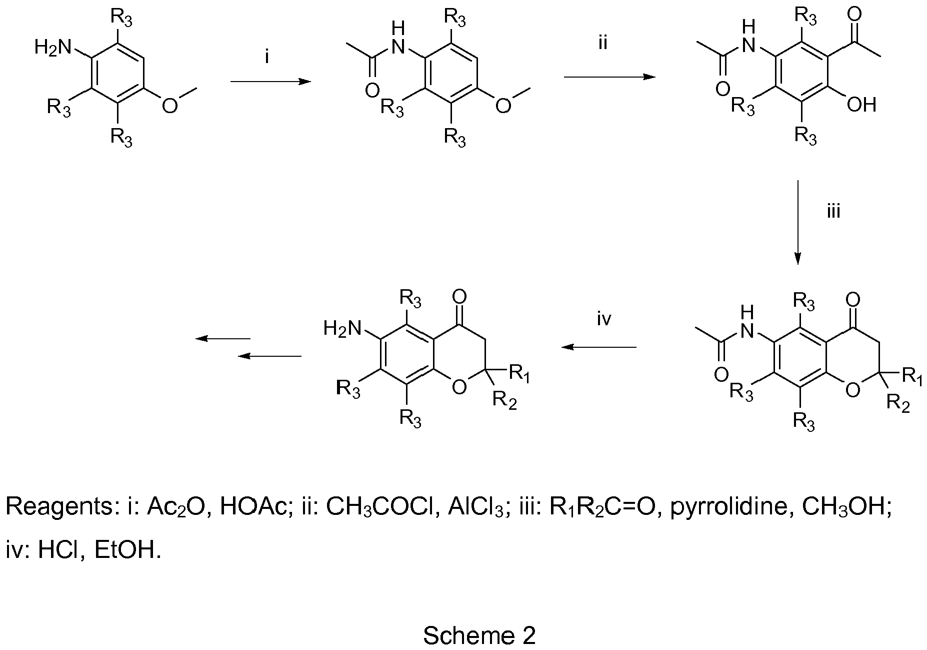

To synthesize the final compounds, the 6-amino-substituted compound obtained after step (iv) of scheme 2 is then engaged in the same sequence of reactions as described for the corresponding 6-amino-substituted compound obtained after step (iii) of scheme 1.

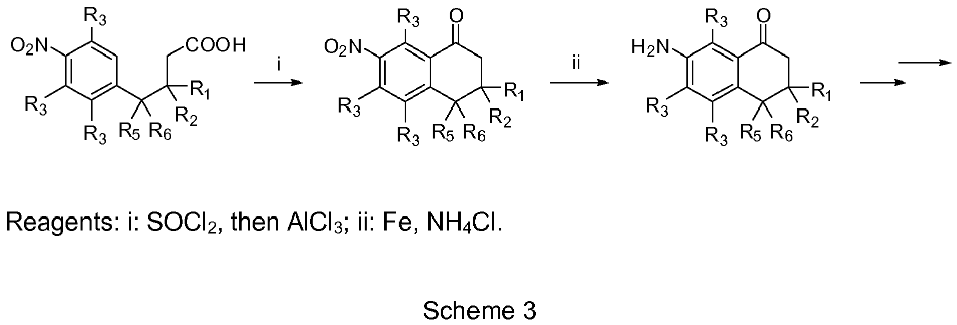

To synthesize the final compounds, the 6-amino-substituted compound obtained after step (ii) of scheme 3 is then engaged in the same sequence of reactions as described for the corresponding 6-amino-substituted compound obtained after step (iii) of scheme 1.

The starting materials are either known compounds or compounds, which may be prepared in analogy with the preparation of known compounds or in analogy with known methods.

### DESCRIPTION OF THE INVENTION

The method of preparing the compound and particularly dihydrobenzopyran, thiochroman, tetrahydroquinoleine and tetrahydronaphthalene derivatives of formula (I) is further illustrated in the following examples, which, however, are not to be construed as limiting.

### Example 1: synthesis of R/S-N-2-chlorophenyl-N'-(6-tert-butoxycarbonylamino-3,4-dihydro-2H-1-benzopyran-4-yl)urea

### 3-(4-nitrophenoxy)propionic acid

A solution of 4-nitrophenol (7 g) in KOH 10% (70 mL) was supplemented with β-bromopropionic acid (7.6 g) and heated at reflux for 5 hours. After cooling, the mixture was acidified by means of 6N HCl and then extracted with ethyl acetate (3 x 100 mL). The organic layers were collected and extracted with a saturated solution of sodium hydrogenocarbonate (3 x 100 mL). The aqueous layers were mixed together and acidified by means of 6N HCl. The resulting precipitate was collected by filtration, washed with water and dried (yield: 40%); melting point: 117-118°C.

### 6-nitro-3,4-dihydro-2H-1-benzopyran-4-one

A solution of 3-(4-nitrophenoxy)propionic acid (4 g) in concentrated sulphuric acid (40 mL) was heated at 50°C for 5 h. The reaction mixture was poured on ice (400 g) and, after complete melting of the ice, extracted with ethyl acetate (3 x 100 mL). The organic layers were collected, dried over anhydrous magnesium sulphate, filtered, and the filtrate was concentrated to dryness. The residue was dissolved in ethyl acetate (25 mL) and the solution was supplemented with hexane (75 mL) under stirring. The resulting precipitate was collected by filtration, washed with hexane and dried (yield: 85%; melting point: 176-178°C.

### 6-amino-3,4-dihydro-2H-1-benzopyran-4-one

A solution of 6-nitro-3,4-dihydro-2H-1-benzopyran-4-one (3 g) in a 3:1 mixture of ethanol and water (60 mL) was heated at 80°C. Ammonium chloride (1.5 g) and iron powder (6 g) were then added and the reaction mixture was heated at 80°C for 15 min. The warm reaction mixture was filtered and the insoluble material was washed with warm ethanol (50 mL). The filtrate was treated with charcoal and, after filtration, was concentrated to dryness. The residue was taken off with water, collected by filtration, washed with water and dried (yield: 70%); melting point: 140-142°C.

### 6-tert-butoxycarbon ylamino-3,4-dihydro-2H-1-benzopyran-4-one

The solution of 6-amino-3,4-dihydro-2*H*-1-benzopyran-4-one (2.5 g) in a 1:1 mixture of THF and water (60 mL) was supplemented with potassium carbonate (4 g) and di-tert-butyl dicarbonate (3.2 g), then the reaction mixture was heated at 40°C for 15 h. After cooling, the organic layer was separated and preserved. The aqueous layer was extracted with ethyl acetate (2 x 50 mL) and the organic layers were collected, dried over magnesium sulphate and filtered. The filtrate was concentrated to dryness and the residue was dissolved in ethyl acetate (20 mL). The solution was supplemented with hexane (60 mL) under stirring and the resulting precipitate was collected by filtration, washed with hexane and dried (yield: 90%); melting point: 146-148°C.

### 6-tert-butoxycarbonylamino-3,4-dihydro-2H-1-benzopyran-4-hydroxyimine

The solution of 6-*tert*-butoxycarbonylamino-3,4-dihydro-2*H*-1-benzopyran-4-one (2.5 g) in ethanol (30 mL) was supplemented with sodium hydrogenocarbonate (2 g) and hydroxylamine hydrochloride (1 g). The reaction mixture was stirred at room temperature during 8 h, then poured on ice. The resulting precipitate was collected by filtration, washed with water and dried (yield: 95%); melting point: 194.5-195.5°C.

### R/S-4-amino-6-tert-butoxycarbonylamino-3,4-dihydro-2H-1-benzopyran

The solution of 6-tert-butoxycarbonylamino-3,4-dihydro-2*H*-1-benzopyran-4-hydroxyimine (2.5 g) in ethanol (25 mL) was supplemented with Raney nickel (3 g). The suspension was introduced in a sealed vessel and submitted to hydrogen gas under pressure (10 bars) during 4 h. The catalyst was eliminated by filtration over celite and the filtrate was evaporated to dryness. The residue was dissolved in methanol (10 mL) and the resulting solution was supplemented with water (80 mL). The precipitate was collected by filtration, washed with water and dried (yield: 70%); melting point: 109-111°C.

### R/S-N-2-chlorophenyl-N'-(6-tert-butoxycarbonylamino-3,4-dihydro-2H-1-benzopyran-4-yl)urea

To the solution of R/S-4-amino-6-*tert*-butoxycarbonylamino-3,4-dihydro-2*H*-1-benzopyran (4.0 mmol) in dichloromethane (5 mL) was added under stirring 2-chlorophenyl isocyanate (4.4 mmol). The reaction mixture was stirred for 30 min and then evaporated to dryness under reduced pressure. The residue was dissolved in the minimum volume of ethyl acetate and then supplemented with two volumes hexane. The resulting precipitate was collected by filtration, washed with hexane and dried (yield: 80%); melting point: 156-159°C.

### Example 2: synthesis of R/S-N-3-chlorophenyl-N'-(6-tert-butoxycarbonylamino-3,4-dihydro-2H-1-benzopyran-4-yl)urea

The title compound was obtained by following the experimental conditions described in example 1 (last step) starting from R/S-4-amino-6-*tert*-butoxycarbonylamino-3,4-dihydro-2*H*-1-benzopyran (1 eq.) and 3-chlorophenyl isocyanate (1.1 eq.); melting point: 133-135°C.

### Example 3: synthesis of R/S-N-4-chlorophenyl-N'-(6-tert-butoxycarbonylamino-3,4-dihydro-2H-1-benzopyran-4-yl)urea

The title compound was obtained by following the experimental conditions described in example 1 (last step) starting from R/S-4-amino-6-*tert*-butoxycarbonylamino-3,4-dihydro-2*H*-1-benzopyran (1 eq.) and 4-chlorophenyl isocyanate (1.1 eq.); melting point: 216-218°C.

### Example 4: synthesis of R/S-N-2-cyanophenyl-N'-(6-tert-butoxycarbonylamino-3,4-dihydro-2H-1-benzopyran-4-yl)urea

The title compound was obtained by following the experimental conditions described in example 1 (last step) starting from R/S-4-amino-6-*tert*-butoxycarbonylamino-3,4-dihydro-2*H*-1-benzopyran (1 eq.) and 2-cyanophenyl isocyanate (1.1 eq.); melting point: 224-227°C.

### Example 5: synthesis of R/S-N-3-cyanophenyl-N'-(6-tert-butoxycarbonylamino-3,4-dihydro-2H-1-benzopyran-4-yl)urea

The title compound was obtained by following the experimental conditions described in example 1 (last step) starting from R/S-4-amino-6-*tert*-butoxycarbonylamino-3,4-dihydro-2*H*-1-benzopyran (1 eq.) and 3-cyanophenyl isocyanate (1.1 eq.); melting point: 212-214°C.

### Example 6: synthesis of R/S-N-4-cyanophenyl-N'-(6-tert-butoxycarbonylamino-3,4-dihydro-2H-1-benzopyran-4-yl)urea

The title compound was obtained by following the experimental conditions described in example 1 (last step) starting from R/S-4-amino-6-*tert*-butoxycarbonylamino-3,4-dihydro-2*H*-1-benzopyran (1 eq.) and 4-cyanophenyl isocyanate (1.1 eq.); melting point: 204-206°C.

### Example 7: synthesis of R/S-N-3-cyanoiphenyl-N'-(6-tert-butoxycarbonylamino-1-thiochroman-4-yl)urea

### 3-(4-nitrophenylsulfanyl)propionic acid

The solution of 4-nitrothiophenol (10 g) in a 10% aqueous solution of KOH (100 mL) was supplemented with β-bromopropionic acid (11 g), and the solution was refluxed for 5 h. After cooling, the reaction mixture was extracted with ethyl acetate (10 mL) and the aqueous layer was separated and treated with 6N HCl until pH 1. The resulting precipitate was collected by filtration, washed with water and dried (yield: 94%); melting point: 129-130°C.

### 6-nitrothiochroman-4-one

The solution of 3-(4-nitrophenylsulfanyl)propionic acid (6 g) in thionyl chloride (60 mL) was refluxed for 4 h. The reaction mixture was evaporated to dryness and the residue was dissolved in anhydrous dichloromethane (60 mL). The solution was supplemented with aluminium chloride (5 g) and the reaction mixture was stirred for 4 h at room temperature. After careful addition of water (60 mL) to the mixture, the organic layer was collected. Then, the aqueous layer was extracted twice with dichloromethane (2 x 50 mL) and the three combined organic layers were dried over anhydrous magnesium sulphate, filtered and evaporated to dryness under reduced pressure. The residue was dissolved in ethyl acetate (25 mL) and the resulting solution was mixed with hexane (75 mL). The precipitate was collected by filtration, washed with hexane and dried (yield: 75%); melting point: 168-170°C.

### 6-aminothiochroman-4-one

The solution of 6-nitrothiochroman-4-one (3 g) in a 3:1 mixture of ethanol and water (60 mL) was heated at 80°C and then supplemented with ammonium chloride (1.5 g) and iron (6 g). After 15 min, the hot reaction mixture was filtered and the insoluble material was washed with hot ethanol (50 mL). The filtrate was treated with charcoal, filtered and then concentrated to dryness. The residue was suspended in water, collected by filtration, washed with water and dried (yield: 86%); melting point: 119-121°C.

### 6-tert-butoxycarbonylaminothiochroman-4-one

The solution of 6-aminothiochroman-4-one (2.5 g) in a 1:1 mixture of THF and water was supplemented with potassium carbonate (4 g) and di-*tert*-butyl dicarbonate (3.2 g) and the reaction mixture was heated at 40°C for 15 h. After cooling, the organic phase was collected and the aqueous phase was extracted twice with ethyl acetate (2 x 50 mL). The three organic phases were combined and dried over anhydrous magnesium sulphate, then filtered and evaporated to dryness. The residue was dissolved in ethyl acetate (20 mL) and mixed with hexane (60 mL). The resulting precipitate was collected by filtration, washed with hexane and dried (yield: 96%); melting point: 138-140°C.

### 6-tert-butoxycarbonylaminothiochroman-4-hydroxyimine

The solution of 6-*tert*-butoxycarbonylaminothiochroman-4-one (2.5 g) in ethanol (30 mL) was supplemented with sodium hydrogenocarbonate (2 g) and hydroxylamine hydrochloride (1 g). The reaction mixture was stirred at room temperature for 8 h and then poured on ice. The resulting precipitate was collected by filtration, washed with water and dried (yield: 95%); melting point: 192-194°C.

### RIS-4-amino-6-tert-butoxycarbonylaminothiochroman

To the solution of 6-*tert*-butoxycarbonylaminothiochroman-4-hydroxyimine (2.5 g) in ethanol (25 mL) was added Raney Nickel (3 g) and the resulting suspension was submitted to hydrogen gas (10 bars) in a sealed vessel at room temperature during 24 h. The catalyst was then removed by filtration over celite and the filtrate was evaporated to dryness. The residue was dissolved in methanol (10 mL) and mixed with water (80 mL). The resulting precipitate was collected by filtration, washed with water and dried (yield: 70%). The crude product was used in the next step without further purification.

### R/S-N-3-cyanophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 3-cyanophenyl isocyanate (1.1 eq.); melting point: 185-187°C.

### Example 8: synthesis of R/S-N-3-chlorophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 3-chlorophenyl isocyanate (1.1 eq.); melting point: 189-191°C.

### Example 9: synthesis of R/S-N-4-chlorophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 4-chlorophenyl isocyanate (1.1 eq.); melting point: 125-128°C.

### Example 10: synthesis of R/S-N-2-chlorophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 2-chlorophenyl isocyanate (1.1 eq.); melting point: 208-210°C.

### Example 11: synthesis of R/S-N-4-cyanophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 4-cyanophenyl isocyanate (1.1 eq.); melting point: 126-130°C.

### Example 12: synthesis of R/S-N-2-cyanophenyl-N'-(6-tert-butoxycarbonylaminothiochroman-4-yl)urea

The title compound was obtained by following the experimental conditions described in example 1 (last step) starting from R/S-4-amino-6-*tert-*butoxycarbonylaminothiochroman (1 eq.) and 2-cyanophenyl isocyanate (1.1 eq.); melting point: 189-191°C.

### Example 13: synthesis of R/S-N-4-cyanophenyl-N'-(6-tert-butoxycarbonylamino-1,1-dioxo-thiochroman-4-yl)urea

### 6-tert-butoxycarbonylamino-1,1-dioxo-thiochroman-4-one

The solution of 6-tert-butoxycarbonylamino-1,1-dioxo-thiochroman-4-one (2.5 g) in dichloromethane (30 mL) was cooled at 0°C and then supplemented with mCPBA (10 g). After 10 min, water (30 mL) was added and the reaction mixture was alcalized by means of a 10% aqueous solution of NaOH. The organic phase was collected and the aqueous phase was extracted twice with ethyl acetate (2 x 50 mL). The three organic phases were combined, dried over anhydrous magnesium sulphate, filtered and evaporated to dryness. The residue was dissolved in ethyl acetate (25 mL) and mixed with hexane (75 mL). The resulting precipitate was collected by filtration, washed with hexane and dried (yield: 83%); melting point: 179-182°C.

### 6-tert-butoxycarbonylamino-1,1-dioxo-thiochroman-4-hydroxyimine

The title compound was obtained by following the experimental conditions described in example 7 for the synthesis of 6-*tert*-butoxycarbonylamino-thiochroman-4-hydroxyimine starting from 6-*tert*-butoxycarbonylamino-1,1-dioxo-thiochroman-4-one; melting point: 163-165°C.

### R/S-4-amino-6-tert-butoxycarbonylamino-1,1-dioxo-thiochroman

The title compound was obtained by following the experimental conditions described in example 7 for the synthesis of R/S-4-amino-6-*tert*-butoxycarbonylaminothiochroman starting from 6-*tert*-butoxycarbonylamino-1,1-dioxo-thiochroman-4-hydroxyimine.

### R/S-N-4-cyanophenyl-N'-(6-tert-butoxycarbonylamino-1,1-dioxo-thiochroman-4-yl)urea

The title compound was obtained by following the experimental conditions described in example 1 (last step) starting from R/S-4-amino-6-*tert*-butoxycarbonylamino-1,1-dioxo-thiochroman (1 eq.) and 4-cyanophenyl isocyanate (1.1 eq.); melting point: 160-162°C.

### Example 14: synthesis of R/S-N-3-cyanophenvl-N'-(6-tert-butoxycarbonylamino-3,4-dihydro-2,2-dimethyl-2H-1-benzopyran-4-yl)urea

### N-(4-methoxyphenyl)acetamide

To the solution of sodium acetate (10.24 g) in acetic acid (41 mL) was added 4-methoxyaniline (50 g) and the reaction mixture was cooled at 0°C. Then acetic anhydride (45.05 mL) was added dropwise under stirring. After 30 min, the reaction mixture was supplemented under stirring with water (100 mL) and the crystalline precipitate of the title compound was collected by filtration. The product was crystallized in hot water and, after cooling, collected by filtration, washed with water and dried (yield: 92%): melting point: 126-127 °C.

### N-(3-acetyl-4-hydroxyphenyl)acetamide

To the suspension of N-(4-methoxyphenyl)acetamide (20 g) in carbon disulfide (50 mL) and acetyl chloride (25 mL) was added aluminum chloride (55 g) portionwise. The reaction mixture was heated at 80-90°C for 90 min, then evaporated under reduced pressure. The residue was suspended in water and ice and the insoluble material was collected by filtration. The product was dissolved in a 5% aqueous solution of sodium hydroxide. The solution was treated with charcoal, filtered and acidified with 12N HCl. The precipitate was collected by filtration, washed with water and dried (yield: 90%); melting point: 162-164°C.

### 6-acetamido-3,4-dihydro-2,2-dimethyl-2H-1-benzopyran-4-one

The solution of N-(3-acetyl-4-hydroxyphenyl)acetamide (25 g) in the mixture of pyrrolidine (15 mL), acetone (32 mL) and methanol (575 mL) was stirred at room temperature overnight. Then, the reaction mixture was evaporated under reduced pressure and the residue was suspended in water. The suspension was adjusted to pH 1 by means of 12N HCl and the insoluble material was then collected by filtration. The precipitate was dissolved in methanol and the resulting solution was treated with charcoal, filtered. The filtrate was mixed with water, collected by filtration, washed with water and dried (yield: 91 %); melting point: 164-165°C.

### 6-amino-3,4-dihydro-2,2-dimethyl-2H-1-benzopyran-4-one

The solution of 6-acetamido-3,4-dihydro-2,2-dimethyl-2*H*-1-benzopyran-4-one (20 g) in a 5N HCl ethanolic solution (250 mL) was refluxed for 3 h. The reaction mixture was mixed with water (500 mL) and the title compound was precipitated by the addition of a 40% aqueous solution of sodium hydroxide until basic pH. The insoluble material was collected by filtration, washed with water and dried (yield: 95%); melting point: 148-149 °C.

### 6-tert-butoxycarbonylamino-3,4-dihydro-2,2-dimethyl-2H-1-benzopyran-4-one

The solution of 6-amino-3,4-dihydro-2,2-dimethyl-2*H*-1-benzopyran-4-one (15 g) in a 1:1 mixture of THF and water (385 mL) was supplemented with potassium carbonate (21.9 g) and di-*tert*-butyl dicarbonate (20.1 g). The reaction mixture was stirred at room temperature for 4 days. The organic layer was collected and the aqueous phase was extracted with ethyl acetate (3 x 100 mL). The organic phases were combined, dried over anhydrous magnesium sulphate, filtered and concentrated to dryness under reduced pressure. The residue was dissolved in methanol and the title compound precipitated by addition of two volumes of water. The precipitate was collected by filtration, washed with water and dried (yield: 95%); melting point: 168-170°C.

### 6-tert-butoxycarbonylamino-3,4-dihydro-2,2-dimethyl-2H-1-benzopyran-4-hydroxyimine

The title compound was obtained by following the experimental conditions described in example 7 for the synthesis of 6-*tert*-butoxycarbonylamino-thiochroman-4-hydroxyimine starting from 6-tert-butoxycarbonylamino-3,4-dihydro-2,2-dimethyl-2*H-*1-benzopyran-4-one (yield: 94%); melting point: 162-163°C.

### R/S-4-amino-6-tert-butoxycarbonylamino-3,4-dihydro-2,2-dimethyl-2H-1-benzopyran

The title compound was obtained by following the experimental conditions described in example 7 for the synthesis of R/S-4-amino-6-*tert*-butoxycarbonylaminothiochroman starting from 6-*tert*-butoxycarbonylamino-3,4-dihydro-2,2-dimethyl-2*H-*1-benzopyran-4-hydroxyimine (yield: 88%); melting point: 144-146°C.

### R/S-N-3-cyanophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-cyanophenyl isocyanate (1.1 eq.); melting point: 191-193°C.

### Example 15: R/S-N-4-cyanophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-cyanophenyl isocyanate (1.1 eq.); melting point: 231-233°C.

### Example 16: R/S-N-3-chloroiphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-chlorophenyl isocyanate (1.1 eq.); melting point: 113°C (decomp.).

### Example 17: R/S-N-4-chlorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-chlorophenyl isocyanate (1.1 eq.); melting point: 113°C (decomp.).

### Example 18: R/S-N-4-fluorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-fluorophenyl isocyanate (1.1 eq.); melting point: 208-210°C.

### Example 19: R/S-N-3-fluorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-fluorophenyl isocyanate (1.1 eq.); melting point: 115-125°C.

### Example 20: R/S-N-3-bromophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-bromophenyl isocyanate (1.1 eq.); melting point: 210-211°C.

### Example 21: R/S-N-3,5-difluorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3,5-difluorophenyl isocyanate (1.1 eq.); melting point: 120-155°C.

### Example 22: R/S-N-4-nitrophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-nitrophenyl isocyanate (1.1 eq.); melting point: 233-234°C.

### Example 23: R/S-N-3-nitrophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-nitrophenyl isocyanate (1.1 eq.); melting point: 120-155°C.

### Example 24: R/S-N-4-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-trifluoromethylphenyl isocyanate (1.1 eq.); melting point: 186-188°C.

### Example 25: R/S-N-3-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3-trifluoromethylphenyl isocyanate (1.1 eq.); melting point: 140-155°C.

### Example 26: R/S-N-3,5-dichlorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2H-1-benzopyran (1 eq.) and 3,5-dichlorophenyl isocyanate (1.1 eq.); melting point: 142-155°C.

### Example 27: R/S-N-2-nitrophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-nitrophenyl isocyanate (1.1 eq.); melting point: 218-219°C.

### Example 28: R/S-N-2-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-trifluoromethylphenyl isocyanate (1.1 eq.); melting point: 239-240°C.

### Example 29: R/S-N-3,5-di(trifluoromethyl)phenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 3,5-di(trifluoromethyl)phenyl isocyanate (1.1 eq.); melting point: 186-190°C.

### Example 30: R/S-N-2-chloro-4-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-chloro-4-trifluoromethylphenyl isocyanate (1.1 eq.); melting point: 234-235°C.

### Example 31: R/S-N-2-chloro-5-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-chloro-5-trifluoromethylphenyl isocyanate (1.1 eq.); melting point: 233-234°C.

### Example 32: R/S-N-2-chloro-6-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-chloro-6-trifluoromethylphenyl isocyanate (1.1 eq.); melting point: 243-244°C.

### Example 33: R/S-N-4-chloro-3-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-chloro-3-trifluoromethylphenyl isocyanate (1.1 eq.); melting point: 213-214°C.

### Example 34: R/S-N-4-bromophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-bromophenyl isocyanate (1.1 eq.); melting point: 181-184°C.

### Example 35: R/S-N-2-chlorophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-chlorophenyl isocyanate (1.1 eq.); melting point: 192-194°C.

### Example 36: R/S-N-2-cyanophenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 2-cyanophenyl isocyanate (1.1 eq.); melting point: 206-208°C.

### Example 37: R/S-N-4-chloro-2-trifluoromethylphenyl-N'-(6-tert-butoxycarbonylamino-3,4-dihydro-2,2-dimethyl-2H-1-benzopyran-4-yl)urea

The title compound was obtained by following the experimental conditions described in example 1 (last step) starting from R/S-4-amino-6-*tert*-butoxycarbonylamino-3,4-dihydro-2,2-dimethyl-2*H*-1-benzopyran (1 eq.) and 4-chloro-2-trifluoromethylphenyl isocyanate (1.1 eq.); melting point: 217-219°C.

### BRIEF DESCRIPTION OF FIGURES

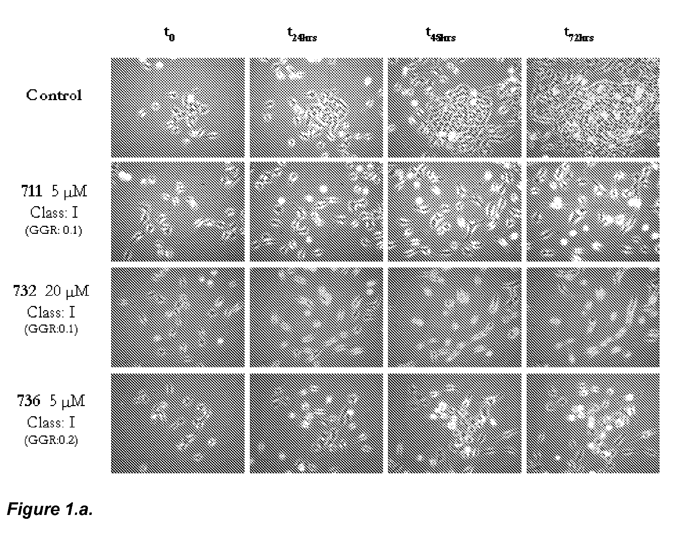

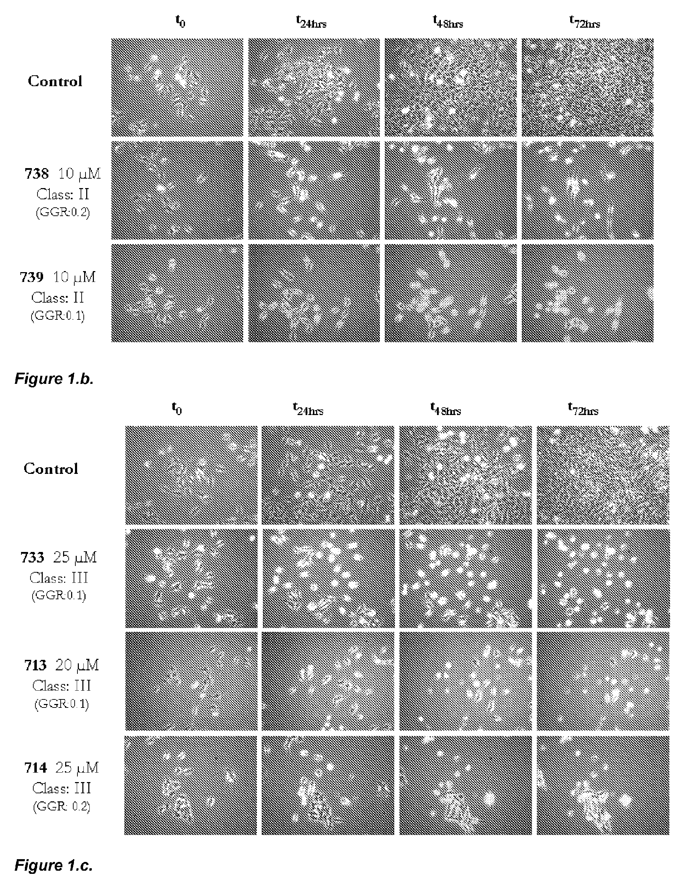

Figure 1a,b,c depict illustrative phase contrast pictures (the videomiscroscopy related pictures) obtained in an *in vitro* cellular imaging approach on human U373 glioblastoma cells untreated or treated with compounds according to the invention. Cellular imaging: pictures (time= 0h, 24h, 48h, 72h) of each U373 cell line left untreated or treated with each compound according to the invention at their IC₅₀ growth inhibitory concentration.
   Fig 1a illustrates the videomiscroscopy related pictures of U373 glioblastoma cells left untreated (control) or treated with compound of example 14 (711), compound of example 19 (732), compound of example 23 (736) (class I) at their IC₅₀ growth inhibitory concentration (see table 1).
   Fig 1b illustrates the videomiscroscopy related pictures of U373 glioblastoma cells left untreated (control) or treated with compound of example 25 (738), compound of example 26 (739) (class II) at their IC₅₀ growth inhibitory concentration (see table 1).
   Fig 1c illustrates the videomiscroscopy related pictures of U373 glioblastoma cells left untreated (control) or treated with compound of example 20 (733), compound of example 16 (713), compound of example 17 (714) (class III) at their IC₅₀ growth inhibitory concentration (see table 1).

### PHARMACOLOGICAL METHOD

The compounds and particularly dihydrobenzopyran, thiochroman, tetrahydroquinoleine and tetrahydronaphthalene derivatives of the invention were evaluated for their anti-proliferative effect (by means of the colorimetric MTT assay), in the following human glioblastoma cell-lines U373, T98G and Hs683 and in the mouse glioma cell line GL261. Normal brain cells (primocultures from newborn mice) were also used in order to determine the bioselectivity (**B^{N/C}**) of the compounds of interest, i.e. the ratio between the concentrations that inhibits 50% of the growth of brain cancer (**C**) cells (glioma cells) and the concentration that inhibits 50% of the growth of normal (**N**) brain cells (primocultures of astrocytes). The ideal situation to be reached is a bioselectivity of B^{N/C} > 10.

The inventors have unexpectedly found that the compounds of the invention appear to have important anti-cancer effects at a given concentration, while not impairing normal cell biology of non-tumor or non-cancer cells at said concentration.

In addition, the inventors could establish that the compounds of the invention act through a non-apoptosis-related mechanism thanks to a unique experimental approach used by the inventors, i.e. computer-assisted phase-contrast microscopy (quantitative videomicroscopy), making them good candidates as anti-cancer drugs for treating apoptosis-resistant tumor or cancer cells and for overcoming problems linked to the known anti-cancer drugs.

### In vitro characterization of the biological effects of the compounds according to the invention

### A/ Effect on overall cell growth

MTT tests were performed in order to rapidly, i.e. within 5 days, measure the effect of compounds of this invention on the overall cell growth. The test measured the number of metabolically active living cells that were able to transform the yellow product 3-(4,5-dimethylthiazol-2-yl)-2,5-diphenyl tetrazolium bromide (herein referred as MTT) into the blue product formazan dye by mitochondrial reduction. The amount of formazan obtained at the end of the experiment, measured by means of a spectrophotometer, is directly proportional to the number of living cells. Optical density determination thus enabled a quantitative measurement of the effect of the investigated compounds as compared to the control condition (untreated cells) and/or to other reference compounds.

Three human cancer cell lines were used in the following MTT tests.

To perform the assay, cells were allowed to grow in 96-well micro-wells with a flat bottom with an amount of 100 µl of cell suspension per well with 1,000 to 8,000 cells/well depending on the cell type used. Each cell line was seeded in its appropriate culture medium.

The detailed experimental procedure was the following: after a 24-hour period of incubation at 37°C, the culture medium was replaced by 100 µl of fresh medium in which the tested compound was previously dissolved, at the following molar concentrations: 10⁻⁸ M, 5.10⁻⁸ M, 10⁻⁷ M, 5.10⁻⁷ M, 10⁻⁶ M, 5.10⁻⁶ M, 10⁻⁵ M, 5.10⁻⁵ M and 10⁻⁴ M. Each experiment was performed in sextuplicate (6 times).

After 72 hours of incubation at 37°C with (experimental conditions) or without (control condition) the compound to be tested, the medium was replaced by 100 µl MTT dissolved in RPMI (1640 without phenol red) at a concentration of 0.5 mg/ml. The micro-wells were subsequently incubated during 3 hours and a half at 37°C and centrifuged at 1300 rpm during 10 minutes. MTT was removed and formazan crystals formed were dissolved in 100 µl DMSO. The micro-wells were shaken for 5 minutes and read on a spectrophotometer at wavelengths of 570 nm (maximal formazan absorbance).

For each experimental condition, the mean optical density was calculated, allowing the determination of the percentage of remaining living cells in comparison to the control.

Table 1 shows the IC₅₀ (representing the range of concentration of the compound tested that resulted in a 50% inhibition of overall tumour cells growth) for each compound in each cell line investigated.

The origin of all the cell lines we used, along with their biological characteristics, and the validation procedures of the MTT colorimetric assay as employed here are fully detailed in Van Quaquebeke et al., J Med Chem 48:849-856, 2005; Ingrassia et al., J Med Chem 52:1100-1114, 2009; Mathieu et al., J Cell Mol Med, Feb. 20, 2009; Maes W et al., Neuro-Oncol 11:529-542, 2009.

**Table 1: IC₅₀ (µM) values: Concentration of compound needed in order to inhibit cell population growth by 50%**

| **Compounds Example n°** | **IC₅₀ (µM) *In Vitro* Growth Inhibitory Concentrations (MTT Colorimetric Assay) Determined in three Human Glioma Cell Lines** | | | |
|---|---|---|---|---|
| | **U373** | **T98G** | **Hs683** | **Mean ± SEM** |
| **14** | 2 | 3 | 2 | 2 ± 1 |
| **29** | 4 | 5 | 3 | 4 ± 1 |
| **23** | 1 | 7 | 16 | 8 ± 4 |
| **26** | 6 | 12 | 9 | 9 ± 2 |
| **21** | 6 | 14 | 7 | 9 ± 3 |
| **24** | 10 | 15 | 11 | 12 ± 1 |
| **25** | 9 | 21 | 8 | 13 ± 4 |
| **27** | 12 | 22 | 13 | 16 ± 3 |
| **16** | 18 | 20 | 12 | 17 ± 3 |
| **15** | 10 | 26 | 18 | 18 ± 5 |
| **34** | 22 | 21 | 21 | 21 ± 1 |
| **33** | 21 | 23 | 22 | 22 ± 1 |
| **19** | 20 | 26 | 19 | 22 ± 2 |
| **20** | 22 | 26 | 18 | 22 ± 2 |
| **31** | 15 | 38 | 12 | 22 ± 8 |
| **22** | 24 | 26 | 20 | 23 ± 2 |
| **1** | 26 | 24 | 23 | 24 ± 1 |
| **17** | 22 | 26 | 26 | 25 ± 1 |
| **2** | 19 | 29 | 28 | 25 ± 3 |
| **30** | 26 | 34 | 25 | 28 ± 3 |
| **5** | 29 | 38 | 25 | 31 ± 4 |
| **18** | 30 | 33 | 35 | 33 ± 1 |
| **37** | 34 | 38 | 30 | 34 ± 2 |
| **6** | 42 | 39 | 30 | 41 ± 2 |
| **4** | 53 | 65 | 44 | 54 ± 6 |
| **36** | 45 | 67 | 54 | 55 ± 6 |
| **32** | 60 | 60 | 66 | 62 ± 2 |
| **35** | 59 | 100 | 34 | 64 ± 19 |
| **3** | 11 | >100 | >100 | >70 |

**Table 2: Bioselectivity determined by dividing the IC₅₀ obtained with glioma cell lines by the IC₅₀ obtained with normal astrocytes. Bioselectivity was determined only for those compounds whose mean + SEM values on the three human glioma cell lines were ≤ 20 µM.**

| **Compounds** | ***In vitro* anti-cancer activity and bioselectivity (Mean IC₅₀ MTT values ± SEM (µM))** | | |
|---|---|---|---|
| **Example n°** | **Glioma - Human Brain cancer cells (I - 72h)** | **Astrocytes - Mouse normal brain cells (II - 72h)** | **Bioselectivity (I / II)** |
| **14** | 2 ± 1 | > 20 | > 10 |
| **29** | 4 ± 1 | ∼ 20 | ∼ 5 |
| **23** | 8 ± 4 | < 40 | < 5 |
| **26** | 9 ± 2 | ∼ 40 | < 5 |
| **21** | 9 ± 3 | ∼ 45 | ∼ 5 |
| **24** | 12 ± 1 | > 120 | > 10 |
| **25** | 13 ± 4 | ∼ 20 | < 2 |
| | | | |
| **16** | 17 ± 3 | > 200 | >12 |
| **15** | 18 ± 5 | > 200 | >10 |

### B/ Impairment of cell proliferation and cell migration triggered by the compound of the present invention in cancer cells

As the MTT test is based on the mitochondria functions, we also investigated the effects of the compounds according to the invention on cell proliferation, migration and morphology by means of a cellular imaging approach (Debeir et al., Cytometry 60:29-40, 2004; Debeir et al., IEEE Trans Med Imaging 24:697-711, 2005) in the human U373 glioma cell line, which is apoptosis-resistant but autophagy-sensitive (Lefranc F et al., Neurosurgery 2008).

Cellular imaging relied on the use of computer-assisted phase-contrast microscopy making possible to film the behaviour of living cells in culture dishes for several days.

Cells were seeded in a 25-cm² flask at a low density, treated or not with the compounds of the invention (at their IC₅₀ concentration determined previously by MTT tests) and filmed thereafter for a period of 72h. The experiment was conducted in triplicate.

The behaviour of the cells, in terms of morphology, growth and death were thus investigated. The effect on the overall growth was measured by counting the number of cells on the first (0h) and the last image (72h) of each film. The global growth ratio (GGR) was then deduced by dividing the number of cells on the last image by the number of cells on the first image. The ratio GGR_{treated cells} / GGR_{control cells}, was further calculated thereby obtaining a value that describes the effect of compounds of the present invention on the overall cell growth.

The methodology is fully described and validated in Debeir et al., 2008, Exp Cell Res 314:2985-2998 and Mathieu et al., 2009, J Cell Mol Med, Feb 20, 2009.

The recordings (not shown) and data obtained clearly show that the compounds according to the invention impair cell morphology and proliferation of human cancer cells. Illustrative pictures (time= 0h, 24h, 48h and 72h) of glioblastoma left untreated or treated with compounds according to the invention (IC₅₀ values calculated by means of the MTT colorimetric assay; see Table 1) are provided in **Figure 1****.**
(**Figure 1**).

Thanks to the quantitative videomicroscopy (QVM) approach, we classified the compounds into 4 distinctive classes on the basis of their cytostatic versus cytotoxic effects (**Table 3**).
- **Class I:** actually cytostatic for 3 days (< 10 % of cell death at the 3^{rd} day)
- **Class II:** cytostatic but becomes cytotoxic with < 10% cell death on the 2^{nd} day and > 20% of cell death on the 3^{rd} day
- **Class III:** cytostatic but rapidly becomes cytotoxic with < 10% cell death on the 1^{st} day, > 20% of cell death on the 2^{nd} day and > 50% of cell death on the 3^{rd} day
- **Class IV:** cytotoxic (> 50% of cell death on the first day)

In Fig. 1a, compounds of example 14 (5 µM), example 19 (20 µM) and example 23 (5 µM) belong to QVM Class I because they display cytostatic effects on human U373 GBM cells during the whole duration of the experiment. Cytostatic effects mean that < 10 % of cell death was recorded at the end of the experiment, i.e. at the end of the 3^{rd} day. QVM analyses enable the global growth ratio (GGR) to be calculated in each experiment. The GGR index is obtained by dividing the number of cells in the last image by the number of cells in the first image of the experiment, knowing that 1080 images are taken during the 72 h-period of observation. The ratio GGR^{treated cells} / GGR^{control cells} was further calculated thereby obtaining a value that describes the effect of compounds on the overall cell growth. Thus, a GGR index of 0.2 means that they remain only 20% of treated cells compare to the control cells at the end of the experiment.

In Fig. 1b, compounds of example 25 (10 µM) and example 26 (10 µM) belong to the QVM Class II because they display cytostatic effects on human U373 GBM cells but this effect becomes cytotoxic on the third day of experiment with < 10% cell death on the 2nd day and > 20% of cell death on the 3rd day.

In Fig. 1c, compounds of example 20 (25 µM), example 16 (20 µM), example 17 (25 µM) belong to the QVM Class III because they are cytostatic but they become rapidly cytotoxic with < 10% cell death on the 1 st day, > 20% of cell death on the 2nd day and > 50% of cell death on the 3rd day.

**Table 3. Quantitative videomicroscopy classification on the basis of the compound cytotoxicity**

| Example n° | QVM Class |
|---|---|
| 14 | I |
| 29 | I |
| 23 | I |
| 26 | II |
| 21 | I |
| 24 | I |
| 25 | II |
| 27 | I |
| 16 | III |
| 15 | I |
| 19 | I |
| 20 | III |
| 31 | II |
| 22 | IV |
| 17 | III |
| 30 | II |
| 18 | I |
| 32 | III |
| 33 | III |
| 34 | III |
| 36 | I |
| 37 | II |

## Claims

1. A compound of general formula (I) 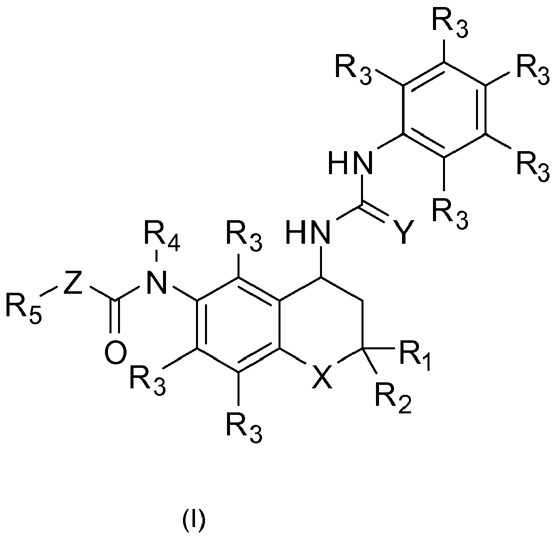 wherein
Y represents Oxygen or Sulphur;
Z represents Oxygen or Sulphur or NH, but if Z represents NH, then R₄ is different from H;
X represents Oxygen, Sulphur or an element selected from the group consisting of (-NR₅, -CR₅R₆ or -SO₂); wherein R₅ and R₆ which can be identical or different, denotes each independently one element selected from the group consisting of (hydrogen, a C₁₋₁₂-alkyl optionally mono- or polysubstituted with a halogen atom or a C₃₋₈-cycloalkyl optionally mono- or polysubstituted with a halogen atom), but if Z represents Oxygen or Sulphur, then R₅ is not hydrogen;
R₁, R₂ and R₄ represent a hydrogen atom, a C₁₋₁₂-alkyl optionally mono- or polysubstituted with a halogen atom or a C₃₋₈-cycloalkyl group optionally mono- or polysubstituted with a halogen atom;
R₁ and R₂ optionally form together a hydrocarbon ring of 3 to 6 atoms, one carbon atom optionally being replaced by an oxygen atom.
R₃ represents an element selected from the group consisting of (hydrogen, halogen, C₁₋₁₂-alkyl optionally mono- or polysubstituted with a halogen atom, C₃₋₈-cycloalkyl optionally mono- or polysubstituted with a halogen atom, hydroxy, C₁₋₆-alkoxy, C₁₋₆-alkoxy-C₁₋₆-alkyl, nitro, amino, cyano, cyanomethyl, perhalomethyl, C₁₋₆-monoalkyl- or dialkylamino, sulfamoyl, C₁₋₆-alkylthio, C₁₋₆-alkylsulfonyl, C₁₋₆-alkylsulfinyl, formyl, C₁₋₆-alkylcarbonylamino, R₇arylthio, R₇arylsulfinyl, R₇arylsulfonyl, C₁₋₆-alkoxycarbonyl, C₁₋₆-alkoxycarbonyl-C₁₋₆-alkyl, carbamoyl, carbamoylmethyl, C₁₋₆-monoalkyl- or dialkylaminocarbonyl, C₁₋₆-monoalkyl-or dialkylaminothiocarbonyl, ureido, C₁₋₆-monoalkyl-or dialkylaminocarbonylamino, thioureido, C₁₋₆-monoalkyl- or dialkylaminothiocarbonylamino, C₁₋₆-monoalkyl- or dialkylaminosulfonyl, carboxy, carboxy-C₁₋₆-alkyl, acyl, R₇aryl, R₇arylalkyl, R₇aryloxy),
where R₇ denotes one or several elements selected from the group consisting of (hydrogen, C₁₋₆-alkyl, halogen, hydroxy or C₁₋₆-alkoxy);
or a salt thereof with a pharmaceutically acceptable acid or base, or any optical isomer or mixture of optical isomers, including a racemic mixture or any tautomeric form thereof.

2. The compounds according to claim 1 **characterized in that** -X- is -O- (oxygen) and represent dihydrobenzopyran derivatives.

3. The compounds according to claim 1 **characterized in that** -X- is -S- (sulphur) or -SO₂-, and represent thiochroman derivatives.

4. The compounds according to claim 1 **characterized in that** -X- is -NR₅-, and represent tetrahydroquinoleine derivatives.

5. The compounds according to claim 1 **characterized in that** -X- is -CR₅R₆- and represent tetrahydronaphthalene derivatives.

6. The dihydrobenzopyran derivatives according to claim 2 **characterized in that** R₁ and R₂ are hydrogen.

7. The dihydrobenzopyran derivatives according to claim 6 selected from:
R/S-*N*-2-chlorophenyl-*N*'-(6-*tert*-butoxycarbonylamino-3,4-dihydro-2*H*-1-benzopyran-4-yl)urea;
R/S-*N*-3-chlorophenyl-*N*'-(6-*tert*-butoxycarbonylamino-3,4-dihydro-2*H*-1-benzopyran-4-yl)urea;
R/S-*N*-4-chlorophenyl-*N*'-(6-tert-butoxycarbonylamino-3,4-dihydro-2*H*-1-benzopyran-4-yl)urea;
R/S-*N*-2-cyanophenyl-*N*'-(6-*tert*-butoxycarbonylamino-3,4-dihydro-2*H*-1-benzopyran-4-yl)urea;
R/S-*N*-3-cyanophenyl-*N*'-(6-*tert*-butoxycarbonylamino-3,4-dihydro-2*H*-1-benzopyran-4-yl)urea;
R/S-*N*-4-cyanophenyl-*N*'-(6-*tert*-butoxycarbonylamino-3,4-dihydro-2*H*-1-benzopyran-4-yl)urea;
or any optical isomer or mixture of optical isomers, including a racemic mixture or any tautomeric form thereof.

8. The thiochroman derivatives according to claim 3 selected from:
R/S-*N*-3-cyanophenyl-*N*'-(6-*tert*-butoxycarbonylaminothiochroman-4-yl)urea;
R/S-*N*-3-chlorophenyl-*N*'-(6-*tert*-butoxycarbonylaminothiochroman-4-yl)urea;
R/S-*N*-4-chlorophenyl-*N*'-(6-*tert*-butoxycarbonylaminothiochroman-4-yl)urea;
R/S-*N*-2-chlorophenyl-*N*'-(6-*tert*-butoxycarbonylaminothiochroman-4-yl)urea;
R/S-*N*-4-cyanophenyl-*N*'-(6-*tert*-butoxycarbonylaminothiochroman-4-yl)urea;
R/S-*N*-2-cyanophenyl-*N*'-(6-*tert*-butoxycarbonylaminothiochroman-4-yl)urea;
R/S-*N*-4-cyanophenyl-*N*'-(6-*tert*-butoxycarbonylamino-1,1-dioxo-thiochroman-4-yl)urea;
or a salt thereof with a pharmaceutically acceptable acid or base, or any optical isomer or mixture of optical isomers, including a racemic mixture or any tautomeric form thereof.

9. The dihydrobenzopyran derivatives according to claim 2 **characterised in that** R₁ and R₂ are methyl.

10. The dihydrobenzopyran derivatives according to claim 9 selected from:
R/S-*N*-3-cyanophenyl-*N*'-(6-*tert*-butoxycarbonylamino-3,4-dihydro-2,2-dimethyl-2H-1-benzopyran-4-yl)urea;
R/S-N-4-cyanophenyl-N'-(6-tert-butoxycarbonylamino-3,4-dihydro-2,2-dimethyl-2H-1-benzopyran-4-yl)urea;
R/S-N-3-chlorophenyl-N'-(6-tert-butoxycarbonylamino-3,4-dihydro-2,2-dimethyl-2*H*-1-benzopyran-4-yl)urea;
R/S-*N*-4-chlorophenyl-*N*'-(6-*tert*-butoxycarbonylamino-3,4-dihydro-2,2-dimethyl-2H-1-benzopyran-4-yl)urea;
R/S-N-4-fluorophenyl-N'-(6-tert-butoxycarbonylamino-3,4-dihydro-2,2-dimethyl-2H-1-benzopyran-4-yl)urea;
R/S-*N*-3-fluorophenyl-*N*'-(6-*tert*-butoxycarbonylamino-3,4-dihydro-2,2-dimethyl-2*H*-1-benzopyran-4-yl)urea;
R/S-*N*-3-bromophenyl-*N*'-(6-*tert*-butoxycarbonylamino-3,4-dihydro-2,2-dimethyl-2*H*-1-benzopyran-4-yl)urea;
R/S-*N*-3,5-difluorophenyl-*N*'-(6-*tert*-butoxycarbonylamino-3,4-dihydro-2,2-dimethyl-2*H*-1-benzopyran-4-yl)urea;
R/S-*N*-4-nitrophenyl-*N*'-(6-*tert*-butoxycarbonylamino-3,4-dihydro-2,2-dimethyl-2*H*-1-benzopyran-4-yl)urea;
R/S-*N*-3-nitrophenyl-*N*'-(6-*tert*-butoxycarbonylamino-3,4-dihydro-2,2-dimethyl-2*H*-1-benzopyran-4-yl)urea;
R/S-*N*-4-trifluoromethylphenyl-*N*'-(6-*tert*-butoxycarbonylamino-3,4-dihydro-2,2-dimethyl-2*H*-1-benzopyran-4-yl)urea;
R/S-*N*-3-trifluoromethylphenyl-*N*'-(6-*tert*-butoxycarbonylamino-3,4-dihydro-2,2-dimethyl-2*H*-1-benzopyran-4-yl)urea;
R/S-*N*-3,5-dichlorophenyl-*N*'-(6-*tert*-butoxycarbonylamino-3,4-dihydro-2,2-dimethyl-2*H*-1-benzopyran-4-yl)urea;
R/S-*N*-2-nitrophenyl-*N*'-(6-*tert*-butoxycarbonylamino-3,4-dihydro-2,2-dimethyl-2*H*-1-benzopyran-4-yl)urea;
R/S-*N*-2-trifluoromethylphenyl-*N*'-(6-*tert*-butoxycarbonylamino-3,4-dihydro-2,2-dimethyl-2*H*-1-benzopyran-4-yl)urea;
R/S-*N*-3,5-di(trifluoromethyl)phenyl-*N*'-(6-*tert*-butoxycarbonylamino-3,4-dihydro-2,2-dimethyl-2*H*-1-benzopyran-4-yl)urea;
R/S-*N*-2-chloro-4-trifluoromethylphenyl-*N*'-(6-*tert*-butoxycarbonylamino-3,4-dihydro-2,2-dimethyl-2*H*-1-benzopyran-4-yl)urea;
R/S-*N*-2-chloro-5-trifluoromethylphenyl-*N*'-(6-*tert*-butoxycarbonylamino-3,4-dihydro-2,2-dimethyl-2*H*-1-benzopyran-4-yl)urea;
R/S-*N*-2-chloro-6-trifluoromethylphenyl-*N*'-(6-*tert*-butoxycarbonylamino-3,4-dihydro-2,2-dimethyl-2*H*-1-benzopyran-4-yl)urea;
R/S-*N*-4-chloro-3-trifluoromethylphenyl-*N*'-(6-*tert*-butoxycarbonylamino-3,4-dihydro-2,2-dimethyl-2*H*-1-benzopyran-4-yl)urea;
R/S-*N*-4-bromophenyl-*N*'-(6-*tert*-butoxycarbonylamino-3,4-dihydro-2,2-dimethyl-2*H*-1-benzopyran-4-yl)urea;
R/S-*N*-2-chlorophenyl-*N*'-(6-*tert*-butoxycarbonylamino-3,4-dihydro-2,2-dimethyl-2*H*-1-benzopyran-4-yl)urea;
R/S-*N*-2-cyanophenyl-*N*'-(6-*tert*-butoxycarbonylamino-3,4-dihydro-2,2-dimethyl-2*H*-1-benzopyran-4-yl)urea;
R/S-*N*-4-chloro-2-trifluoromethylphenyl-*N*'-(6-*tert*-butoxycarbonylamino-3,4-dihydro-2,2-dimethyl-2*H*-1-benzopyran-4-yl)urea;
or a salt thereof with a pharmaceutically acceptable acid or base, or any optical isomer or mixture of optical isomers, including a racemic mixture or any tautomeric form thereof.

11. The dihydrobenzopyran derivative which is R/S-*N*-3-cyanophenyl-*N*'-(6-*tert-*butoxycarbonylamino-3,4-dihydro-2,2-dimethyl-2*H*-1-benzopyran-4-yl)urea, or any optical isomer or mixture of optical isomers, including a racemic mixture or any tautomeric form thereof.

12. A compound according to any one of claim 1 to 11 for use as a medicament.

13. A compound according to any one of claim 1-11 for use in the treatment or prevention of proliferative disorder, preferably cancer.

14. The compound according to claim 13 for use in the treatment or prevention of brain cancer.

15. A pharmaceutical composition comprising a compound according to any one of the preceding claims 1 to 11 or pharmaceutically acceptable salt thereof with a pharmaceutically acceptable acid or base or any optical isomer or mixture of optical isomers, including a racemic mixture or any tautomeric form together with one or more pharmaceutically acceptable carriers of diluents.

16. A pharmaceutical composition according to claim 15 for use in the treatment or prevention of proliferative disorder.

17. The pharmaceutical composition according to claim 16 for use in the treatment or prevention of cancer preferably brain cancer.

18. A process for the manufacture of a medicament, particular to be use in the treatment of proliferative disorder, which process comprising bringing a compound of formula (I) according to any one of the preceding claims 1 to 11 or a pharmaceutically acceptable salt thereof into a galenic dosage form.
